# EUROPÄISCHE PATENTSCHRIFT

(11) **EP 3 081 244 B1**
(45) Veröffentlichungstag und Bekanntmachung des Hinweises auf die Patenterteilung: **22.08.2018**
(21) Anmeldenummer: 16164810.0
(22) Anmeldetag: 12.04.2016
(51) Int. Cl.: A61M 1/02

(54) **VORRICHTUNG ZUR SAMMLUNG UND VERARBEITUNG VON MENSCHLICHEM BLUT**
DEVICE FOR COLLECTING AND PROCESSING HUMAN BLOOD
DISPOSITIF DE COLLECTE ET DE TRAITEMENT DE SANG HUMAIN

(30) Priorität: 16.04.2015 DE 202015101866 U
(43) Veröffentlichungstag der Anmeldung: 19.10.2016
(73) Patentinhaber: Meise, Heinz, 58579 Schalksmühle (DE)
(72) Erfinder: Meise, Heinz, 58579 Schalksmühle (DE)
(74) Vertreter: Patentanwälte Dörner & Kötter PartG mbB

(56) Entgegenhaltungen:
- EP-A1- 2 604 245
- WO-A1-2014/016198
- US-A1- 2014 234 183

## Beschreibung

Die Erfindung betrifft eine Vorrichtung zur Sammlung und Verarbeitung von menschlichem Blut oder Blutbestandteilen.

Die Sammlung und Verarbeitung von menschlichem Blut oder Blutbestandteilen erfolgt üblicherweise in einer Vorrichtung, in der mindestens ein Beutel Verwendung findet, an dem ein Schlauch angeschlossen ist. Es können jedoch auch zwei, drei oder auch vier Beutel über ein Schlauchsystem miteinander in Verbindung stehen. Zudem können an dem Beutel-Schlauch-System zusätzlich Probenentnahmeeinrichtungen oder andere notwendige Einrichtungen vorhanden sein, die dann ebenfalls über Schläuche mit den Beuteln in Verbindung stehen.

Die Sammlung des menschlichen Bluts oder der Blutbestandteile erfolgt in Blutspenden, bei denen das Blut mittels einer Kanüle aus der Arterie eines Menschen gesogen wird. Üblicherweise wird das Blut in einem sog. Spendebeutel, der Bestandteil eines Blutbeutelsystems ist, gesammelt. Die übrigen Beutel und Einrichtungen sind fest mit dem Spendebeutel verbunden. Nach der Spende wird das gesamte Beutelsystem in eine Zentrifuge eingestellt, in der es in einer Art Bechersystem bei bis zu 4.000 G zentrifugiert wird. In diesem Vorgang wird in dem Spendebeutel das Plasma von den zellulären Bestandteilen getrennt.

Nach der Zentrifugierung befindet sich in dem Spendebeutel im oberen Bereich das Plasma; im unteren Bereich befinden sich die roten zellulären Bestandteile; dazwischen eine Mischung aus Thrombozyten und Leukozyten, welches gemeinhin als "Buffy-Code" bezeichnet wird. Der Spendebeutel wird im Anschluss in eine Trennvorrichtung eingespannt, in der sich eine Presse befindet. Die Presse sorgt dafür, dass das in dem Spendebeutel oben befindliche Plasma nach oben aus dem Beutel herausgepresst wird, die roten zellulären Bestandteile, die "Erythrozyten", dagegen nach unten aus dem Beutel gepresst werden. Die so getrennten Bestandteile werden in weitere Beutel gefördert und weiterverarbeitet.

Die bekannte Art der Behandlung des Blutes hat sich grundsätzlich bewährt. Allerdings weisen die bekannten Blut-Schlauch-Systeme den Nachteil auf, dass sie auch als Zweifach-, Dreifach- oder auch Vierfach-Beutelsysteme den gesamten Prozess durchlaufen müssen. Infolgedessen ist die Handhabung erschwert. So ist es zum Beispiel notwendig, bereits bei der Blutentnahme am Spender bis zu vier Beutel plus Filtern und Zusatzeinrichtungen zu handhaben. Darüber hinaus müssen immer alle Beutel plus Zusatzeinrichtungen in den Zentrifugen untergebracht werden. Dadurch ist beispielsweise der in den Zentrifugen benötigte Raum für die Aufnahme eines Beutel-Schlauch-Systems erhöht.
Vor diesem Hintergrund ist es wünschenswert, so wenig Beutel wie möglich bei den einzelnen Behandlungsschritten handhaben zu müssen. So ist es zum Beispiel während der Blutspende ausreichend, wenn lediglich ein Sammelbeutel benutzt werden muss. Auch bei der Zentrifugation reicht es aus, wenn nur der das Blut enthaltende Beutel in der Zentrifuge behandelt wird. Um dies zu ermöglichen, ist es beispielsweise bekannt, Beutel nur mit einem Schlauch zu versehen und vor der Weiterverarbeitung einen weiteren Beutel, mittels einer Schweißverbindung anzuschließen. Dies erfolgt mit Hilfe von Sterilschweißgeräten. Solche Schweißgeräte weisen eine heiße Kupferklinge auf, die die Schlauchenden steril verschweißt. Zwar ist dies grundsätzlich eine Möglichkeit, Schläuche von Blutbeutel-Schlauchsystemen miteinander zu verbinden; allerdings ist es erforderlich, die Klinge nach jedem Schweißvorgang auszutauschen, was einerseits kostenintensiv ist, andererseits auch Bedienfehler beinhalten kann. Auch besteht die Gefahr, dass das Wechseln der Klinge vom Personal unterlassen wird, so dass nicht in jedem Falle gewährleistet werden kann, dass die erforderliche Sterilität erreicht ist.
Beutel-Schlauch-Systeme zur Trennung von Blutbestandteilen sind beispielsweise aus der US 2014/0234183 A1 und der EP 2 604 245 A1 bekannt. Darüber hinaus ist aus der WO 2014/016198 A1 ein System zur mittels Schwerkraft erfolgenden Aufteilung von Spenderblut bekannt, bei dem ein Konnektor zur Verbindung von Schlauchteilen zur Anwendung kommt. Die Schlauchteile sind bis zur ihrer Verbindung durch abnehmbare Kappen verschlossen sind, die vor dem Verbinden der Schlauchteile abgenommen werden. Damit ist jedoch keine sterile Verbindung der Schlauchteile möglich.

Hier will die Erfindung Abhilfe schaffen. Der Erfindung liegt die Aufgabe zu Grunde, eine Vorrichtung zur Sammlung und Verarbeitung von menschlichem Blut oder Blutbestandteilen zu schaffen, die einerseits die Handhabung wesentlich vereinfacht und andererseits eine sterile Verbindung von Schläuchen gewährleistet. Gemäß der Erfindung wird diese Aufgabe mit den Merkmalen des Schutzanspruchs 1 gelöst.

Mit der Erfindung ist eine Vorrichtung zur Sammlung und Verarbeitung von menschlichem Blut oder Blutbestandteilen geschaffen, welche die Handhabung und auch die Verbindung von Schläuchen wesentlich vereinfacht. Dies findet seine Ursache in der Verwendung der Kupplungsteile, welche aufgrund ihrer Korrespondenz einfach ineinander fügbar sind. Die Kupplungen ermöglichen es dem Bedienpersonal in einfacher Weise, zwei Schlauchenden miteinander zu verbinden, so dass ein geschlossenes System geschaffen ist. Gleichzeitig kann auf die Verwendung von Sterilschweißgeräten verzichtet werden, was einerseits Bedienfehler reduziert, und andererseits zu einer Kostenersparnis beiträgt.

Dabei sind die Kupplungsteile gasdicht verschlossen. Dies ermöglicht die einfache Handhabung der erfindungsgemäßen Vorrichtung Durch den gasdichten Verschluss ist verhindert, dass die im Bereich der Gewinnung und Verarbeitung von menschlichem Blut erforderliche Sterilität verletzt wird.

Der Verschluss der Kupplungsteile erfolgt erfindungsgemäß mit Membranen. Diese Ausgestaltung hat sich bewährt, da die Membranen unmittelbar in die Kupplungsteile eingebracht werden können und somit optimal in den Herstellprozess eingebunden werden können. Es bedarf daher keiner externen Abdichtung der Kupplungsteile.

Andere Weiterbildungen und Ausgestaltungen der Erfindung sind in den übrigen Unteransprüchen angegeben. Ein Ausführungsbeispiel der Erfindung ist in der Zeichnung dargestellt und wird nachfolgend im Einzelnen beschrieben. Es zeigen:

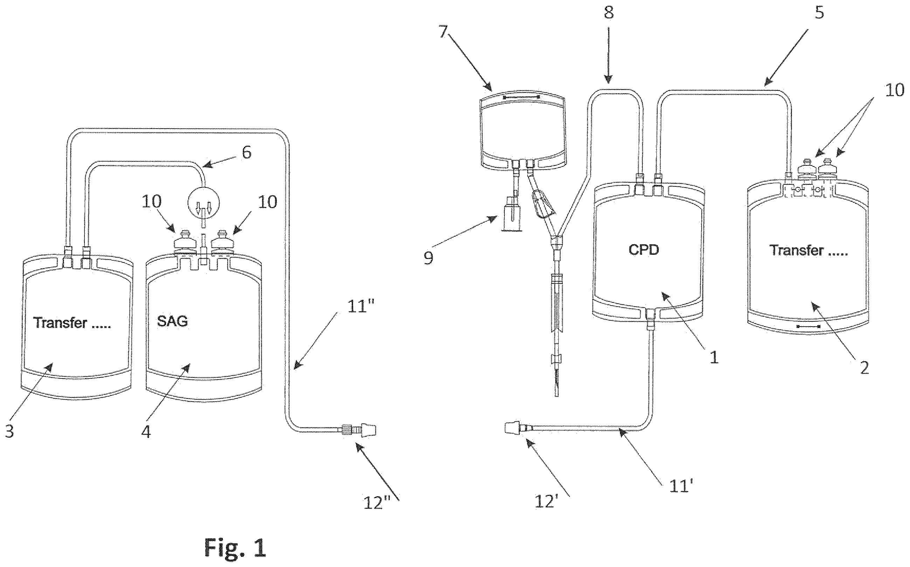

- Figur 1:: die schematische Darstellung eines Blutbeutel-Schlauch-Systems mit vier Beuteln;

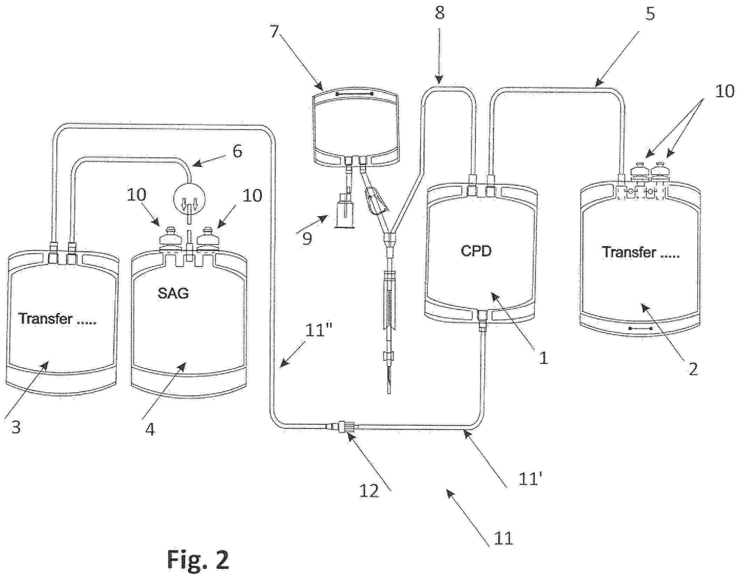

- Figur 2: das in Figur 1 dargestellte Blutbeutel-Schlauch-System mit geschlossener Kupplung.

Bei der als Ausführungsbeispiel gewählten Vorrichtung zur Sammlung und Verarbeitung von menschlichem Blut oder Blutbestandteilen kommen vier Blutbeutel 1, 2, 3, 4 zur Anwendung. Die Blutbeutel 1 und 2 sind über einen Schlauch 5 miteinander verbunden und bilden ein erstes Beutelsystem. Die Beutel 3 und 4 sind über einen Schlauch 6 miteinander verbunden und bilden ein zweites Beutelsystem. An dem Beutel 1 ist zudem ein Probenentnahmebeutel 7 über einen Schlauch 8 angeschlossen. Der Probenentnahmebeutel 7 ist mit einer Probeentnahmevorrichtung 9 versehen, an die ein Probeentnahme-Röhrchen anschließbar ist.

Die Beutel 1, 2, 3, 4 sind aus Kunststoff hergestellt. In der Regel findet eine Schlauchfolie zur Herstellung der Beutel Anwendung, die innen aufgeraut ist, so dass ein Aneinanderkleben der Wände der Beutel verhindert ist. An ihren Stirnseiten ist die Folie verklebt, wodurch die Beutel entstehen. Andere Arten der Herstellung der Beutel und die Verwendung anderer Materialien sind selbstverständlich möglich. Die Schläuche sind aus einem Kunststoff hergestellt, der eine Sterilisation ermöglicht.

Die Schläuche 5, 6 und 8 sind an den Beuteln 1, 2, 3, 4 nicht lösbar befestigt. An den Beuteln 2 und 4 sind zudem an einer Stirnseite jeweils Entnahmestutzen 10 angeordnet, die dicht verschlossen sind. Die Entnahmestutzen 10 können durch Drehen oder Knicken aktiviert werden.

Das erste Beutelsystem ist über einen Schlauch 11 mit dem zweiten Beutelsystem verbindbar. Der Schlauch 11 besteht aus den beiden Schlauchteilen 11' und 11". An dem Boden des Beutels 1 ist das Schlauchteil 11' angeschlossen.

Ebenso ist an dem Beutel 3 das Schlauchteil 11" angeschlossen, das an der Kopfseite des Beutels vorgesehen ist. Die Schlauchteile 11' und 11" dienen der Verbindung der beiden Beutelsysteme des Ausführungsbeispiels.
Die Schlauchteile 11' und 11" sind an ihren den Beuteln 1 und 3 abgewandten Enden mit Kupplungsteilen 12' und 12" versehen, die ineinanderfügbar sind und gemeinsam eine Kupplung 12 bilden. Die Kupplungsteile 12' und 12" bestehen aus Kunststoff. Sie sind mit den Schlauchteilen 11' und 11" verschweißt. Die Kupplungsteile 12' und 12" ermöglichen eine sterile Verbindung der beiden Beutelsysteme. Hierzu sind in den Kupplungsteilen 12' und 12" geeignete Maßnahmen ergriffen, in Form von Membranen, so dass die Kupplungsteile 12' und 12" und damit auch die Schlauchteile 11' und 11" vor dem Ineinanderfügen gasdicht verschlossen sind. Die Membranen stellen den sterilen Raum innerhalb der Kupplungsteile 12' und 12" bereit. Sie können beispielsweise aus Elastomer oder Silikon bestehen. Sie sind derart ausgebildet, dass während des Zusammensteckens der Kupplungsteile 12' und 12" die Membranen zerstört werden, so dass der Durchfluss von dem einen in das andere Beutelsystem durch die Schlauchteile 11' und 11" sowie die Kupplungsteile 12' und 12" möglich ist. Die Kupplungsteile 12' und 12" können auch derart ausgebildet sein, dass nach der Verbindung der Kupplungsteile 12' und 12" die Membranen aktiv zerstört werden müssen, beispielsweise durch axial bewegliche Teile, die die Membranen durchstoßen und so ebenfalls den Durchfluss von dem einen in das andere Beutelsystem durch die Schlauchteile 11' und 11" sowie die Kupplungsteile 12' und 12" möglich machen.
Bei der Verwendung der erfindungsgemäßen Vorrichtung erfolgt zunächst die Blutentnahme bei dem jeweiligen Spender. Das Blut wird in dem Spendebeutel 1 gesammelt, der in der Regel ein Fassungsvermögen von 500 ml aufweist. Im Anschluss wird es in einer Zentrifuge geschleudert, so dass sich oben gerinnungsaktives Plasma absetzt und unten die Erythrozyten. Nach Einlegen des Spendebeutels 1 in eine Presseinrichtung wird das Plasma nach oben aus dem Beutel 1 durch den Schlauch 5 in den Beutel 2 gedrückt. Der Beutel 2 mit dem Plasma kann dann durch Trennen des Schlauchs 5 von dem Beutel 1 getrennt werden und allein weiterverarbeitet werden. Der Beutel 1, an dessen Boden sich noch die Erythrozyten und darüber der "Buffy-Coat" befinden, wird dann weiterbehandelt, beispielsweise erneut in einer Zentrifuge. In beiden Zentrifugen ist es nunmehr lediglich erforderlich, die Beutel 1 und 2 mit dem Schlauch 5 in der Zentrifuge anzuordnen, und nicht wie im Stand der Technik alle vier Beutel 1 bis 4 mit den zugehörigen Schläuchen.
Die Weiterverarbeitung der im Beutel 1 verbliebenen Bestandteile kann unter Benutzung des Schlauchs 11 erfolgen. Zur Verbindung mit dem weiteren Beutel-Schlauch-System, bestehend aus den Beuteln 3 und 4 sowie dem Schlauch 6, werden in einfacher Weise die Kupplungsteile 12' und 12" ineinander gefügt, wodurch eine dichte und sterile Verbindung der Schlauchteile 11' und 11" und damit des Schlauchs 11 geschaffen ist. Nach Durchstoßen der Membranen ist ein Durchfließen ermöglicht, so dass die Bestandteile aus dem Beutel 1 bei Bedarf in den Beutel 3 gepresst werden kann und der weiteren Verarbeitung unterzogen werden kann.

## Patentansprüche

1. Vorrichtung zur Sammlung und Verarbeitung von menschlichem Blut oder Blutbestandteilen, umfassend ein erstes Beutelsystem aus Beuteln (1, 2), die über einen Schlauch (5) miteinander verbunden sind, und ein zweites Beutelsystem aus Beuteln (3, 4), die über einen Schlauch (6) miteinander verbunden sind, wobei an dem ersten Beutelsystem ein Schlauchteil (11') angeschlossen ist und das Schlauchteil (11') an seinem dem Beutel (1) abgewandten Ende mit einem Kupplungsteil (12') versehen ist, das mit einem zweiten Kupplungsteil (12") korrespondiert, welches an einem Schlauchteil (11") des zweiten Beutelsystems an dem dem Beutel (3) abgewandten Ende vorgesehen ist, und die Kupplungsteile (12', 12") ineinanderfügbar sind und gemeinsam eine Kupplung (12) bilden, wobei in den Kupplungsteilen (12', 12") Membranen vorgesehen sind, die die Schlauchteile (11', 11") und Kupplungsteile (12', 12") gasdicht verschließen und wobei nach Durchstoßen der Membranen ein Durchfließen ermöglicht wird.

2. Vorrichtung nach Anspruch 1, **dadurch gekennzeichnet, dass** das Kupplungsteil (12") an ein zweites Schlauchteil (11") angeschlossen ist.

3. Vorrichtung nach einem oder mehreren der vorgenannten Ansprüche, **dadurch gekennzeichnet, dass** die Kupplungsteile (12', 12") mit den Schlauchteilen (11', 11") verschweißt sind.

## Claims

1. Device for collecting and processing human blood or blood components comprising a first bag system of bags (1, 2) which are connected to one another via a tube (5) and a second bag system of bags (3, 4) which are connected to one another via a tube (6), wherein a tube part (11') is connected to the first bag system and the tube part (11') is provided with a coupling part (12') at its end facing away from the bag (1), which corresponds with a second coupling part (12"), which is provided at a tube part (11") of the second bag system at the end facing away from the bag (3) and the coupling parts (12', 12") can be inserted into one another and together form a coupling (12), wherein membranes are provided in the coupling parts (12', 12") which seal the tube parts (11', 11") and coupling parts (12', 12") in a gastight manner and wherein, after piercing of the membranes, passage is allowed for.

2. Device according to claim 1, **characterised in that** the coupling part (12") is connected to a second tube part (11").

3. Device according to one or more of the previous claims, **characterised in that** the coupling parts (12', 12") are welded to the tube parts (11', 11").

## Revendications

1. Dispositif de collecte et de traitement du sang humain ou de ses constituants, comprenant un premier système de poches (1, 2) reliées entre elles via un flexible (5), et un deuxième système de poches (3, 4) reliées entre elles via un flexible (6), sachant que contre le premier système de poches est raccordé un segment de flexible (11') et que ce segment de flexible (11') est relié, en son extrémité en regardant pas la poche (1), avec une pièce de raccordement (12') qui épouse une deuxième pièce de raccordement (12"), laquelle deuxième pièce est prévue contre un segment de flexible (11") du deuxième système de poches à l'extrémité ne regardant pas la poche (3), et que les pièces de raccordement (12', 12") sont emboîtables les unes dans les autres et forment ensemble un raccord (12), sachant que dans les pièces de raccordement (12', 12") sont prévues des membranes qui obturent les segments de flexibles (11', 11") et les pièces de raccordement (12', 12") de manière étanche aux gaz, et sachant qu'après avoir perforé les membranes le liquide peut circuler.

2. Dispositif selon la revendication 1, **caractérisé en ce que** la pièce de raccordement (12") est raccordée à un deuxième segment de flexible (11").

3. Dispositif selon l'une ou plusieurs des revendications précédentes, **caractérisé en ce que** les pièces de raccordement (12', 12") sont reliées par soudage aux segments de flexibles (11', 11").
